# EUROPÄISCHE PATENTSCHRIFT

(11) **EP 2 582 694 B1**
(45) Veröffentlichungstag und Bekanntmachung des Hinweises auf die Patenterteilung: **22.07.2015**
(21) Anmeldenummer: 11726391.3
(22) Anmeldetag: 10.06.2011
(51) Int. Cl.: C07D 401/14, A01N 43/713, A61P 33/00, A61K 31/4439

(54) **VERFAHREN ZUR HERSTELLUNG VON TETRAZOL-SUBSTITUIERTEN ANTHRANILSÄUREDIAMID-DERIVATEN UND NEUE KRISTALLINE MODIFIKATION DIESER DERIVATE**
PROCESS FOR MANUFACTURING TETRAZOLE- SUBSTITUTED ANTHRANILIC ACID DIAMIDE DERIVATIVES AND NOVEL CRYSTALLINE MODIFICATION OF THESE DERIVATIVES
PROCÉDÉ DE FABRICATION DE DÉRIVÉS DE DIAMIDE D'ACIDE ANTHRANILIQUE SUBSTITUÉS PAR TÉTRAZOLE ET NOUVELLE MODIFICATION CRISTALLINE DE CES DÉRIVÉS

(30) Priorität: 15.06.2010 EP 10166059; 15.06.2010 US 354920 P
(43) Veröffentlichungstag der Anmeldung: 24.04.2013
(60) Teilanmeldung: 15171321.1
(73) Patentinhaber: Bayer Intellectual Property GmbH, 40789 Monheim (DE)
(72) Erfinder: PAZENOK, Sergii, 42699 Solingen (DE); LUI, Norbert, 51519 Odenthal (DE); VOLZ, Frank, 50825 Köln (DE); OLENIK, Britta, 46242 Bottrop (DE); FUNKE, Christian, 42799 Leichlingen (DE); FISCHER, Rüdiger, 50259 Pulheim (DE); GAERTZEN, Oliver, 50931 Köln (DE); HINZ, Martin-Holger, 42499 Hückeswagen (DE); NEEFF, Arnd, 51399 Burscheid (DE)
(86) Internationale Anmeldenummer: PCT/EP2011/059735
(87) Internationale Veröffentlichungsnummer: WO 2011/157664

(56) Entgegenhaltungen:
- WO-A1-2007/144100
- WO-A2-2010/069502
- R. J. SPEAR: "Positional selectivity of the methylation of 5-substituted tetrazolate anions", AUSTRALIAN JOURNAL OF CHEMISTRY, Bd. 37, Nr. 12, 1984, Seiten 2453-2468, XP000653166,
- WILLIAM P. NORRIS: "5-Trifluoromethyltetrazole and Its Derivatives", JOIURNAL OF ORGANIC CHEMISTRY, Bd. 27, Nr. 9, September 1962 (1962-09), Seiten 3248-3251, XP002607646, in der Anmeldung erwähnt
- WILLIAM G. FINNEGAN, RONALD A. HENRY: "Synthesis and Reactions of 1-Nitroso-1-alkyl-2-guanyl-and -2-carbamylhydrazines", JOURNAL OF ORGANIC CHEMISTRY, Bd. 30, Nr. 2, Februar 1965 (1965-02), Seiten 567-575, XP002607647,
- RONALD A. HENRY, WILLIAM G. FINNEGAN: "Mono-alkylation of Sodium 5-Aminotetrazole in Aqueous Medium", JOURNAL OF THE AMERICAN CHEMICAL SOCIETY, Bd. 76, Nr. 3, Februar 1954 (1954-02), Seiten 923-926, XP002607648, in der Anmeldung erwähnt
- CAIRA M R: "CRYSTALLINE POLYMORPHISM OF ORGANIC COMPOUNDS", TOPICS IN CURRENT CHEMISTRY, SPRINGER, BERLIN, DE, Bd. 198, 1. Januar 1998 (1998-01-01), Seiten 163-208, XP001156954, ISSN: 0340-1022, DOI: DOI:10.1007/3-540-69178-2_5 ISBN: 978-3-540-36760-4

## Beschreibung

Die vorliegende Offenbarung beschreibt ein Verfahren zur Herstellung von tetrazol-substituierten Anthranilsäurediamid-Derivaten der Formel (I) 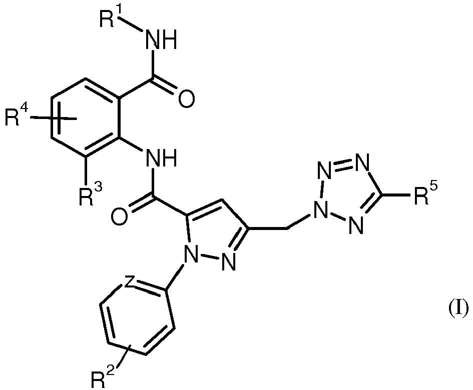 durch Umsetzung von N-Aryl und N-hetarylsubstituierten Pyrazolen, welche Methylentetrazolreste enthalten. Die vorliegende Erfindung betrifft die Bereitstellung einer neuen kristallinen Modifikation der entsprechenden Anthranilsäurediamid-Derivate durch dieses Verfahren, sowie die neue kristalline Modifikation und ihre Verwendung in agrochemischen Zubereitungen.

In der Literatur wurde bereits beschrieben, dass beispielsweise die Alkylierung von Alkyltetrazolen mit zum Beispiel Alkyliodiden üblicherweise zu einer Mischung aus verschiedenen Regioisomeren führt. Darüberhinaus hängt die Zusammensetzung des Gemisches stark von den jeweiligen Substituenten am Tetrazolring ab. So beschreibt William P. Norris, in J. Org. Chem., 1962, 27 (9), 3248-3251, dass bei der Alkylierung von 5-Trifluormethyltetrazol mit Methyljodid eine Mischung von beiden Regioisomeren in Verhältnis 6:1 entsteht. Die Alkylierung von Alkyltetrazolen führt dagegen meistens zu 1-substituerten Tetrazolen (siehe R-A.Henry et al, JACS, 76, 923 (1954). Weiterhin sind mehrere Verfahren zur Herstellung von Anthranilsäureamiden enthalten in WO 21010/069502, wobei Anthranilsäureamide mit unterschiedlichen Tetrazolsubstitutenten erhalten werden können. Die Bereitstellung von neuen kristallinen Modifikationen ist jedoch nicht beschrieben.

Ebenfalls bekannt ist, dass das Auftreten von Wirkstoffen in verschiedenen kristallinen Modifikationen (Polymorphie) sowohl für die Ausarbeitung von Herstellungsverfahren als auch für die Entwicklung von Formulierungen von großer Bedeutung ist. So unterscheiden sich die verschiedenen kristallinen Modifikationen einer chemischen Verbindung neben dem Aussehen (Kristallhabitus) und der Härte auch in zahlreichen weiteren physiko-chemischen Eigenschaften. Dabei können Unterschiede bezüglich der Stabilität, der Filtrierbarkeit, der Löslichkeit, der Hygroskopizität, des Schmelzpunktes, der Feststoffdichte und der Fließfähigkeit einen starken Einfluss auf die Qualität und die Wirksamkeit von Pflanzenbehandlungsmitteln ausüben. Es ist bisher nicht möglich, das Auftreten und die Anzahl von kristallinen Modifikationen einschließlich ihrer physiko-chemischen Eigenschaften vorherzusagen. Vor allem die thermodynamische Stabilität und auch das unterschiedliche Verhalten nach Darreichung in lebenden Organismen lassen sich nicht im Voraus bestimmen.

Die Aufgabe der vorliegenden Erfindung ist die Bereitstellung einer neuen kristallinen Modifikation eines dieser tetrazol-substituierten Anthranilsäurediamid-Derivate, die aufgrund ihrer physikochemischen Eigenschaften gut zu handhaben ist und die Produktion einer stabilen Formulierung ermöglicht.

Beschrieben wird auch ein Verfahren zur Herstellung von Anthranilsäurediamid-Derivaten der allgemeinen Formel (I) 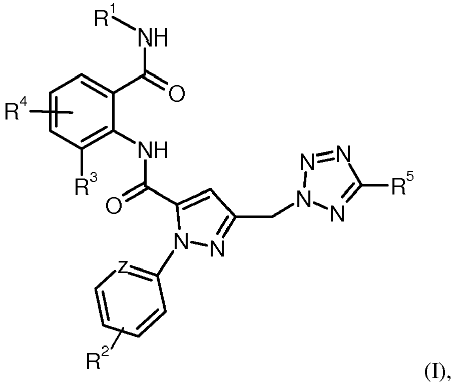 in welcher
- R¹, R³: unabhängig voneinander für Wasserstoff, für gegebenenfalls einfach oder mehrfach, gleich oder verschieden durch Halogen oder Nitro substituiertes C₁-C₆-Alkyl, C₁-C₆-Alkoxy, C₂-C₆-Alkenyl, C₂-C₆-Alkinyl oder C₃-C₆-Cycloalkyl steht, bevorzugt für (C₁-C₅)-Alkyl, besonders bevorzugt für Methyl, Ethyl oder tert-Butyl steht, ganz besonders bevorzugt für Methyl steht,
- R²: für C₁-C₆-Alkyl, C₃-C₆-Cycloalkyl, C₁-C₆-Haloalkyl, C₁-C₆-Halocycloalkyl, C₂-C₆-Alkenyl, C₂-C₆-Haloalkenyl, C₂-C₆-Alkinyl, C₂-C₆-Haloalkinyl, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Haloalkylthio, C₁-C₄-Haloalkylsulfinyl, C₁-C₄-Haloalkylsulfonyl, Halogen, Cyano, Nitro, Alkylamino, Dialkylamino, Cycloalkylamino oder C₃-C₆-Trialkylsilyl steht, bevorzugt für Halogen oder C₁-C₆-Alkyl steht, besonders bevorzugt für Fluor oder Chlor steht, ganz besonders bevorzugt für Chlor steht,
- R⁴: für Wasserstoff, Halogen, Cyano, Nitro C₁-C₄-Alkyl, C₁-C₄-Haloalkyl, C₂-C₆-Alkenyl, C₂-C₆-Haloalkenyl, C₂-C₆-Alkinyl, C₁-C₄-Alkoxy, C₁-C₄-Haloalkoxy, SF₅, C₁-C₄-Alkylthio, C₁-C₄-Alkylsulfinyl, C₁-C₄-Alkylsulfonyl, C₁-C₄-Haloalkylthio, C₁-C₄-Haloalkylsulfinyl, C₁-C₄-Haloalkylsulfonyl, C₁-C₄-Alkylamino, Di-(C₁-C₄-alkyl)amino, C₃-C₆-Cycloalkylamino, (C₁-C₄-Alkoxy)imino, (C₁-C₄-Alkyl)(C₁-C₄-Alkoxy)imino, (C₁-C₄-Haloalkyl)(C₁-C₄-Cyano, Nitro, Alkoxy)imino oder C₃-C₆-Trialkylsilyl steht, bevorzugt für Wasserstoff, Chlor oder Cyano, besonders bevorzugt für Chlor oder Cyano steht, ganz besonders bevorzugt für Cyano steht,
- R⁵: für C₁-C₅-Alkyl, welches 1 bis 3 fach durch Halogen substituiert sein kann, bevorzugt für C₁-C₃ Perfluoralkyl, besonders bevorzugt für CF₃ oder C₂F₅ steht, ganz besonders bevorzugt für CF₃ steht,
- Z: für CH und N steht, bevorzugt für N steht,

die Verbindungen der allgemeinen Formel (I) außerdem N-Oxide und Salze umfassen,
dadurch gekennzeichnet, dass man N-Aryl und N-hetarylsubstituierte Pyrazole der Formel (II) 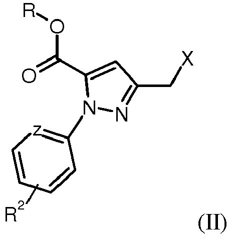 in welcher
- R: für (C₁-C₆) Alkyl, Aryl(C₁-C₆)-Alkyl oder Aryl steht,
R², Z oben angegebenen Bedeutungen haben
und X für Fluor, Chlor, Brom, Jod, CH₃SO₂O, CF₃SO₃ oder p-CH₃-C₆H₄SO₃ steht,
mit Tetrazolen der Formel (III) 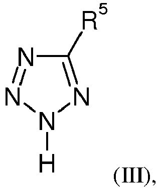 in welcher R⁵ die oben angegebenen Bedeutungen hat,
zu Pyrazolcarbonsäureestern der Formel (IV) umsetzt 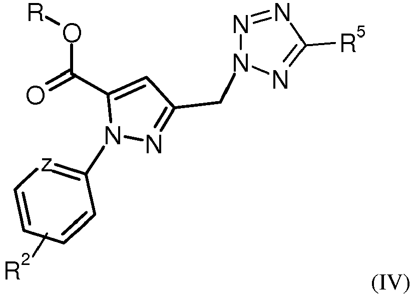 in welcher
R, R², R⁵ und Z oben angegebenen Bedeutungen haben,
und diese gegebenenfalls ohne vorherige Isolierung zu Pyrazolcarbonsäuren der Formel (V) umsetzt 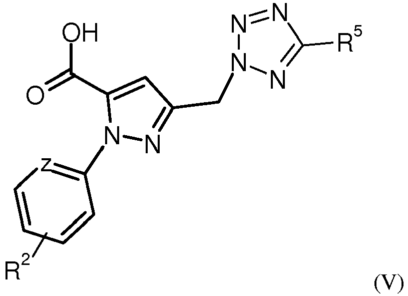 in welcher R², R⁵ und Z oben angegebenen Bedeutungen haben
und diese mit Verbindungen der allgemeinen Formel (VI) 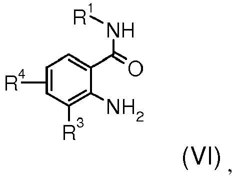 wobei R¹, R³, R⁴ die oben angegebenen Bedeutungen haben,
umsetzt zu Anthranilsäurediamiden der Formel (I), 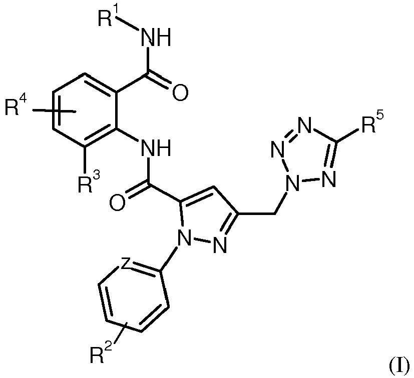 in welcher R¹, R², R³, R⁴, R⁵ und Z oben angegebenen Bedeutungen haben.

Durch das erfinderische Verfahren werden die Verbindungen der Formel (I) mit einer Reinheit von > 90%, bevorzugt von 91 %-97 %, besonders bevorzugt von 93 % bis 97 % erhalten. Das 1-Isomer, welches an der 1-Position des Tetrazolrings gebunden ist, entsteht nur zu 5-10%.

Das Verfahren kann anhand des folgenden Schemas (I) erläutert werden :

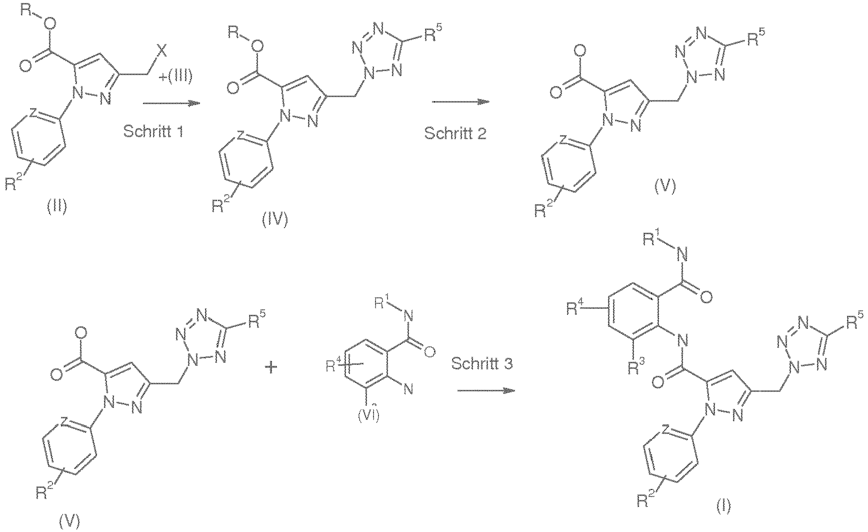

In welcher R¹, R², R³, R, R⁴, R⁵, Z und X die oben angegebenen allgemeinen Bedeutungen haben, wobei R¹ in Formel VI (Schritt 3) nicht für Wasserstoff steht.

### Schema (I)

### Allgemeine Definitionen:

Im Zusammenhang mit der vorliegenden Erfindung umfasst der Begriff Halogene (X), soweit nicht anders definiert, solche Elemente, die ausgewählt sind aus der Gruppe bestehend aus Fluor, Chlor, Brom und Jod, wobei Fluor, Chlor und Brom bevorzugt und Fluor und Chlor besonders bevorzugt verwendet werden. Substituierte Gruppen können einfach oder mehrfach substituiert sein, wobei bei Mehrfachsubstitutionen die Substituenten gleich oder verschieden sein können.

Mit einem oder mehreren Halogenatomen (-X) substituierte Alkyl-Gruppen = (Halogenalkyl-Gruppen) sind beispielsweise ausgewählt aus Trifluormethyl (CF₃), Difluormethyl (CHF₂), CCl₃, CFCl₂, CF₃CH₂, ClCH₂, CF₃CCl₂.

Alkyl-Gruppen sind im Zusammenhang mit der vorliegenden Erfindung, soweit nicht abweichend definiert, lineare oder verzweigte Kohlenwasserstoff-Gruppen.

Die Definition Alkyl und C₁-C₁₂-Alkyl umfasst beispielsweise die Bedeutungen Methyl, Ethyl, n-, isoPropyl, n-, iso-, sec- und t-Butyl, n-Pentyl, n-Hexyl, 1,3-Dimethylbutyl, 3,3-Dimethylbutyl, n-Heptyl, n-Nonyl, n-Decyl, n-Undecyl, n-Dodecyl.

Cycloalkyl-Gruppen sind im Zusammenhang mit der vorliegenden Erfindung, soweit nicht abweichend definiert, ringförmige gesättigte Kohlenwasserstoff-Gruppen.

Aryl-Reste sind im Zusammenhang mit der vorliegenden Erfindung, soweit nicht abweichend definiert, aromatische Kohlenwasserstoff-Reste, die ein, zwei oder mehrere Heteroatome, die ausgewählt sind aus O, N, P und S aufweisen können und optional durch weitere Gruppen substituiert sein können.

Arylalkyl-Gruppen und Arylalkoxy-Gruppen sind im Zusammenhang mit der vorliegenden Erfindung, soweit nicht abweichend definiert, durch Aryl-Gruppen substituierte Alkyl- bzw. Alkoxy-Gruppen, die eine Alkylenkette aufweisen können. Im Einzelnen umfasst die Definition Arylalkyl beispielsweise die Bedeutungen Benzyl- und Phenylethyl-; die Definition Arylalkoxy bespielsweise die Bedeutung Benzyloxy.

Alkylaryl-Gruppen (Alkaryl-Gruppen) und Alkylaryloxy-Gruppen sind im Zusammenhang mit der vorliegenden Erfindung, soweit nicht abweichend definiert, durch Alkyl-Gruppen substituierte Aryl-Gruppen, bzw Aryloxy-Gruppen, die eine C₁₋₈-Alkylenkette aufweisen können und im Arylgerüst oder Aryloxygerüst ein oder mehrere Heteroatome, die ausgewählt sind aus O, N, P und S, aufweisen können.

Die Verbindungen können gegebenenfalls als Mischungen verschiedener möglicher isomerer Formen, insbesondere von Stereoisomeren, wie z.B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen offenbart

Durch das oben beschriebene Verfahren wird eine neue kristalline Modifikation der folgenden Verbindung der Formel (I-1) 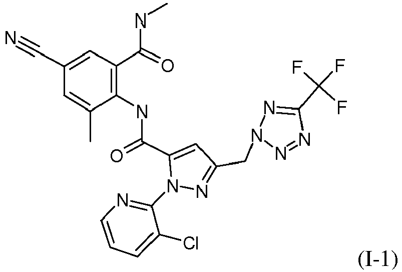 bereitgestellt, die nachstehend als kristalline Modifikation B bezeichnet wird. Dieses Verfahren wid in Anspruch 1 beschrieben und ist Teil der Erfindung.

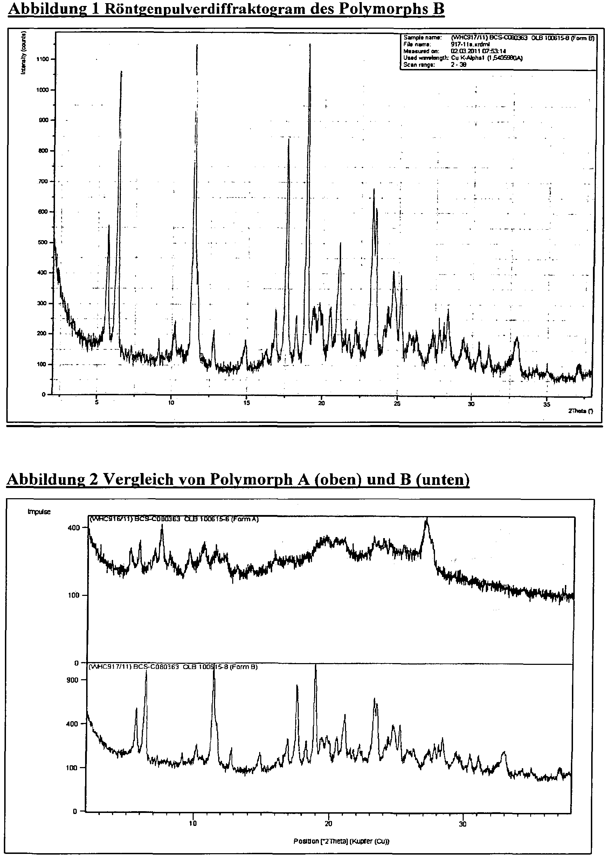

Die Verbindung der Formel (1-1) ist aus WO2010/069502 bekannt. Bislang war von der Verbind (3-Chloropyridin-2-yl)-N-[4-cyan-2-methyl-6-(methylcarbamoyl)phenyl]-3-{[5-(trifluormethyl)-2H-tetrazol-2-yl]methyl}-1H-pyrazol-5-carboxamid der Formel (1-1) nur eine Kristallform bekannt, die im Folgenden als Modifikation A bezeichnet wird. Modifikation A hat ein charakteristisches Röntgenpulverdiffraktogramm, Raman-Spektrum und IR-Spektrum (Tab. 1-2, Abb. 2, 3, 5). Diese kristalline Modifikation ist thermodynamisch metastabil und fällt in Form blockartiger Kristalle an.

Daher wurde die Aufgabe gelöst, durch das erfindungsgemäße Verfahren die neue kristalline Modifikation B bereitzustellen, die aufgrund ihrer physikochemischen Eigenschaften gut zu handhaben ist und die Herstellung einer stabilen Formulierung ermöglicht.

Gegenstand der Erfindung ist daher ebenfalls die kristalline Modifikation B, die dadurch gekennzeichnet ist, dass sie ein Röntgenpulverdiffraktogramm mit den in Tabelle 1 angegebenen Reflexlagen (2 Theta) aufweist. Das Röntgenpulverdiffraktogramm der kristallinen Modifikation B ist auch in der Abbildung 1 wiedergegeben. Die intensivsten Signale (2 Theta) des Röntgenpulver-diffraktogramms der kristallinen Modifikation B liegen demnach bei 5.7°, 6.4°, 11.4°, 17.6°, 18.9°, 21.1°, 23.2°, 23.4°, 23.5° und 25.2° (jeweils ± 0,2°)

Alle Röntgenpulverdiffraktometrie-Daten der Modifikation B wurden mit folgenden Akquisitionsparametern erhalten:

| | |
|---|---|
| Diffraktometer Typ: | PANalytic X'Pert PRO |
| Anodenmaterial: | Cu |
| Wellenlänge: | 1.54060 |
| Scan Modus: | Transmission |
| Scan Typ: | 2Theta:Omega |
| Angabe 2Theta: | ± 0,2° |

Die bekannte kristalline Modifikation A der Verbindung der Formel (1) ist dadurch gekennzeichnet, dass sie ein Röntgenpulverdiffraktogramm mit den in der folgenden Tabelle 1 angegebenen Reflexlagen (2 Theta) aufweist. Das Röntgenpulverdiffraktogramm der kristallinen Modifikation A ist auch in der Abbildung 2 wiedergegeben.

Alle Röntgenpulverdiffraktometrie-Daten der Modifikation A wurden ebenfalls mit folgenden Akquisitionsparametern erhalten:

| | |
|---|---|
| Diffraktometer Typ: | PANalytic X'Pert PRO |
| Anodenmaterial: | Cu |
| Wellenlänge: | 1.54060 |
| Scan Modus: | Transmission |
| Scan Typ: | 2Theta:Omega |
| Angabe 2Theta: | ± 0,2° |

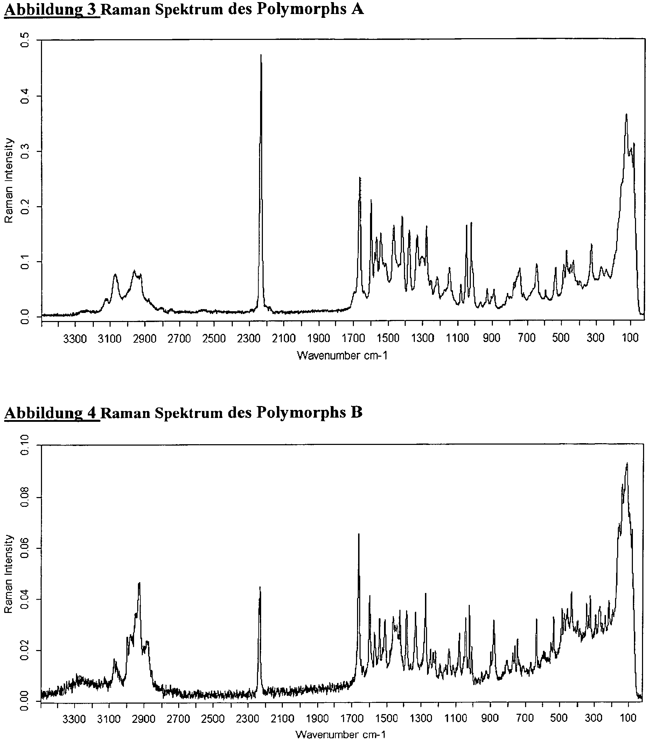

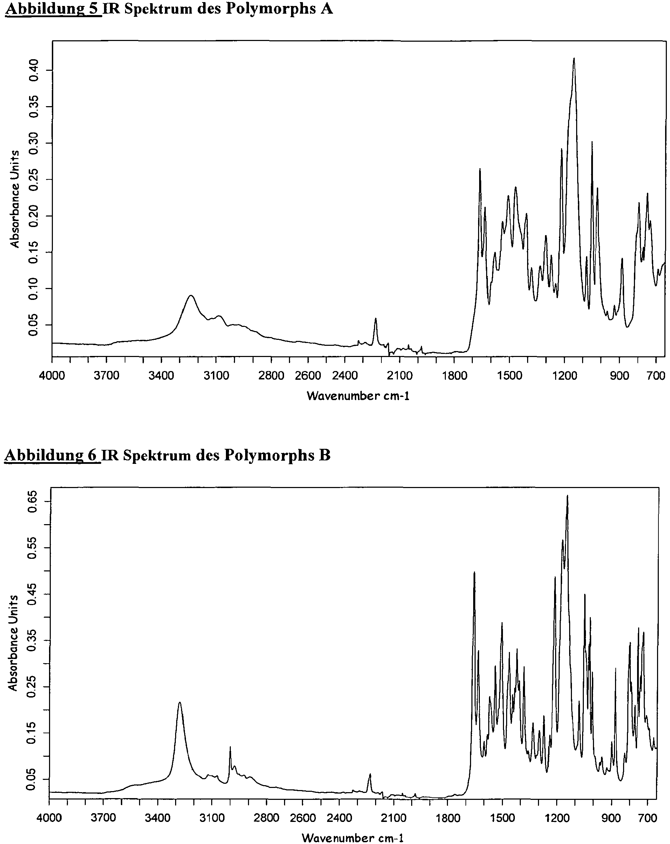

Die erfindungsgemäße kristalline Modifikation B der Verbindung der Formel (1-1) kann außerdem durch IR- und Raman-Spektroskopie charakterisiert werden. Die entsprechenden Raman- und IR-Spektren sind in Abbildung 4 und 6 abgebildet.

Die IR- und Raman-Spektren enthalten folgende Banden der kristallinen Modifikationen A und B, die in Tabelle 2 aufgelistet sind.

Im folgenden wird das erfindungsgemäße Verfahren, durch welches die neue kristalline Modifikation B der Verbindung der Formel (1-1) erhalten wird, näher beschrieben :

### Schritt 1.

Pyrazolcarbonsäureester der Formel (IV) können folgendermaßen hergestellt werden: 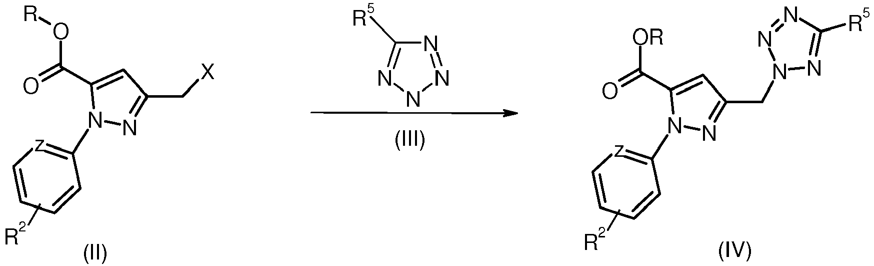 wobei R, R², X, Z, R⁵ die oben angegebenen Bedeutungen haben.

Tetrazole der Formel (III) sind bekannt, teilweise sogar kommerziell erhältlich oder können nach bekannten Verfahren erhalten werden (vgl. z.B. WO2004/020445; William P. Norris, J. Org. Chem., 1962, 27 (9), 3248-3251; Henry C. Brown, Robert J. Kassal, J. Org. Chem., 1967, 32 (6), 1871-1873; Dennis P. Curran, Sabine Hadida, Sun-Young Kim, Tetrahedron, 1999, 55 (29), 8997-9006; L.D. Hansen, E.J. Baca, P. Scheiner, Journal of Heterocyclic Chemistry, 1970, 7, 991-996), JACS V.27, p.3248

Die Alkylierung von Alkyltetrazolen mit zum Beispiel MeJ führt üblicherweise zu einer Mischung aus 1- und 2- substituerten Alkyltetrazolen, wobei die Zusammensetzung stark von derm Alkylierungsreagenz und von den Substiuenten am Pyrazolring abhängig ist. Als überraschend anzusehen ist, dass die Alkylierung von beispielsweise Perfluoralkyltetrazolen der Formel (III) mit Pyrazolen der Formel (II) mit hoher Ausbeute und hoher Selektivität in der Position 2 des Tetrazolrings stattfindet, wobei das 1-Isomer nur in Mengen von 5-10 % entsteht.

5-Trifluormethyltetrazol (R⁵ = CF₃ in Formel (III)) ist eine sehr starke organische Säure mit pKa 1.14, welche nur geringfügig schwächer ist als Trifluoressigsäure. Dagegen ist das Trifluormethylterazolyl-Anion ein sehr schwaches Nukleophil oder Base. Deswegen werden für die Alkylierung von CF₃-Tetrazolen meistens hochreaktive und teure Alkyliodide oder hochreaktive Benzylchloride (siehe W.Finnegan et al. Journal of Organic Chemistry (1965), 30(2), 567-75) eingesetzt.

Die Alkylierung von Tetrazolen mit Halomethylpyrazocarbonsäure-Derivaten der Formel (II) ist nicht bekannt und der Reaktionsverlauf bei Umsetzung mit Perfluoralkyl-Tetrazolen der Formel (III) war nicht abzusehen.

Als überraschend anzusehen ist daher, dass die Alkylierung von Perfluoralkyltetrazolen der Formel (III) mit Pyrazolen der Formel (II) mit hoher Ausbeute und hoher Selektivität in der Position 2 des Tetrazolrings stattfindet, wobei das 1-Isomer nur in Mengen von 5-10 % entsteht. Ausserdem ist es überraschend, dass während der Reaktion zwischen Perfluoralkyltetrazolen der Formel (III) mit beispielsweise Chlormethylpyrazolen der Formel (II) keine Alkylierung des Pyridinrings stattfindet.

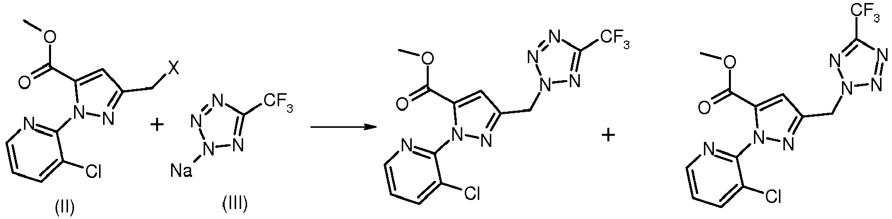

Für die Alkylierung von beispielsweise CF₃-Tetrazolen der Formel (III) mit Chlormethylpyrazolen der Formel (II) (X=Cl) ist es notwendig, die Reaktion durch Verwendung von Katalysator zu beschleunigen. Als Katalysatoren dienen organische und anorganische Bromide wie KBr, CsBr oder bevorzug Jodide wie KJ, NaJ, CsJ, Me₄NI, Bu₄NI.

Überraschenderweise kann durch Anwesenheit der Katalysatoren die Alkylierung mit schwach reaktiven Chlormethylpyrazolen der Formel (II) durchgeführt werden, sodass nicht die teueren Iodide wie in JACS V.27, p.3248 beschrieben, verwendet werden müssen. Dabei wird die Regioselektivität der Reaktion nicht negative beeinflusst. Die Katalysatoren werden in Mengen zwischen 0.1 bis 1 Mol, bevorzugt 0.1-0.5Mol bezogen auf Pyrazol der Formel (II) eingesetzt.

Die Durchführung des erfindungsgemäßen Verfahrensschritts erfolgt vorzugsweise innerhalb eines Temperaturbereichs von 40°C bis +120°C, besonders bevorzugt bei Temperaturen von 40°C bis +80 °C.

Der erfindungsgemäße Verfahrensschritt (1) wird im Allgemeinen unter Normaldruck durchgeführt. Alternativ ist es jedoch auch möglich, im Vakuum zu arbeiten oder unter Druck..

Die Reaktionszeit ist nicht kritisch und kann, in Abhängigkeit von der Ansatzgröße vom Substituent R⁵ im Pyrazolring und von der Temperatur, in einem Bereich zwischen ein und mehreren Stunden gewählt werden.

Bei der Durchführung des erfindungsgemäßen Verfahrensschritts setzt man für 1 Mol des Pyrazoles der Formel (II) 0,8 Mol bis 1,4 Mol, vorzugsweise 0,9 Mol bis 1,2 Mol, besonders bevorzugt 1,1 Mol. des Tetrazols der Formel (III) ein.

Die Reaktion wird grundsätzlich in Gegenwart einer Base durchgeführt. Geeignete Basen sind z. B. Natriumhydroxid, Kaliumcarbonat, Natriumcarbonat, Cäsiumcarbonat, Natriummethylat, Triethylamin oder Natriumhydrid. Bevorzugt wird das Tetrazol der Formel (III) in der Form des Na-Salz eingesetzt.

Geeignete Lösungsmittel sind beispielsweise aliphatische, alicyclische oder aromatische Kohlenwasserstoffe, wie z.B. Petrolether, n-Hexan, n-Heptan, Cyclohexan, Methylcyclohexan, Benzol, Toluol, Xylol oder Decalin, und halogenierte Kohlenwasserstoffe, wie z.B. Chlorbenzol, Dichlorbenzol, Dichlormethan, Chloroform, Tetrachlormethan, Dichlorethan oder Trichlorethan, Ether, wie Diethylether, Diisopropylether, Methyl-tert-butylether, Methyl-tert-amylether, Dioxan, Tetrahydrofuran, 1,2-Dimethoxyethan, 1,2-Diethoxyethan oder Anisol; Nitrile, wie Acetonitril, Propionitril, n- oder iso-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Sulfoxide, wie Dimethylsulfoxid oder Sulfone, wie Sulfolan, Alkohole wie Methanol, Ethanol, Isopropanol. Besonders bevorzugt verwendet man Acetone, Acetonitrile, Toluol, Methyltert.Butylether, THF. Die Trennung von beiden Regioismeren kann durch Chromatographie, Kristallisation erfolgen oder das Gemisch kann ohne Reinigung weiter umgesetzt werden.

Die gebildeten Produkte können ohne vorherige Aufarbeitung im darauffolgenden Schritt (2), in welchem Verseifung stattfindet, eingesetzt werden.

Pyrazolcarbonsäureester-Derivate der Formel (II) sind bekannt oder können nach bekannten Verfahren erhalten werden (vgl. z.B. WO2007/144100).

### Schritt 2

Die im Schritt 1 gebildeten Verbindungen der Formel (IV) werden zu Pyrazolcarbonsäuren der Formel (V) umgesetzt:

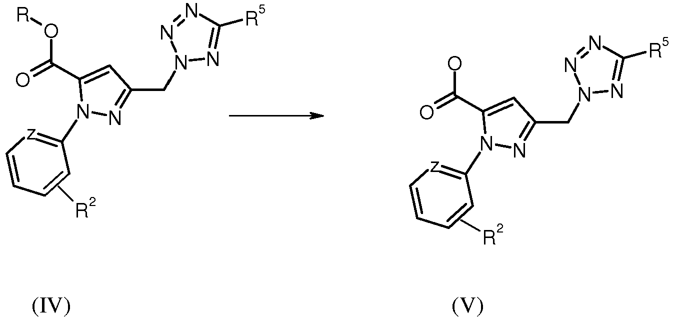

Die Verseifung wird in der Regel unter sauren oder basischen Bedingungen durchgeführt.

Für saure Hydrolyse bevorzugt sind mineralische Säuren, beispielsweise H₂SO₄, HCl, HSO₃Cl, HF, HBr, HI, H₃PO₄ oder organische Säuren, beispielsweise CF₃COOH, p-Toluolsulfonsäure, Methansulfonsäure, Trifluormethansulfonsäure. Die Reaktion kann durch der Zusatz von Katalysatoren wie beispielsweise FeCl₃, AlCl₃, BF₃, SbCl₃, NaH₂PO₄ beschleunigt werden. Die Reaktion kann ebenfalls ohne Zusatz von Säure nur in Wasser durchgeführt werden.

Basische Hydrolyse erfolgt in Gegenwart von anorganischen Basen wie, Alkalimetallhydroxide wie z.B. Lithium-, Natrium- oder Kaliumhydroxid, Alkalimetallcarbonate wie beispielsweise Na₂CO₃, K₂CO₃ und -Acetate wie beispielsweise NaOAc, KOAc, LiOAc, sowie -Alkoholate, wie z.B. NaOMe, NaOEt, NaOt-Bu, KOt-Bu von organischen Basen wie Trialkylamine, Alkylpyridine, Phosphazene und 1,8-Diazabicyclo[5.4.0]undecen (DBU). Bevorzug sind die anorganische Basen wie NaOH, KOH, Na₂CO₃, K₂CO₃.

Die Durchführung des erfindungsgemäßen Verfahrensschritts (2) erfolgt vorzugsweise innerhalb eines Temperaturbereichs von 20°C bis +150°C, besonders bevorzugt bei Temperaturen von 30°C bis +110 °C, besonders bevorzugt bei 30-80°C.

Der erfindungsgemäße Verfahrensschritt (2) wird im Allgemeinen unter Normaldruck durchgeführt. Alternativ ist es jedoch auch möglich, im Vakuum oder unter Überdrück (z.B. Umsetzung im Autoklav mit wässriger HCl ) zu arbeiten.

Die Reaktionszeit kann, in Abhängigkeit von der Ansatzgröße und der Temperatur, in einem Bereich zwischen 1 Stunde und mehreren Stunden gewählt werden.

Der Reaktionsschritt 2 kann in Substanz oder in einem Lösungsmittel durchgeführt werden. Vorzugsweise wird die Reaktion in einem Lösungsmittel durchgeführt. Geeignete Lösungsmittel sind beispielsweise ausgewählt aus der Gruppe bestehend aus Wasser, Alkoholen wie Methanol, Ethanol, Isopropanol oder Butanol, aliphatischen und aromatischen Kohlenwasserstoffen, wie z.B. n-Hexan, Benzol oder Toluol, die durch Fluor- und Chloratome substituiert sein können, wie Methylenchlorid, Dichlorethan, Chlorbenzol oder Dichlorbenzol; Ethern, wie z.B. Diethylether, Diphenylether Methyl-tert-butylether, Isopropylethylether, Dioxan, Diglym, Dimethylglycol, Dimethoxyethane (DME) oder THF; Nitrilen wie Methylnitril, Butylnitril oder Phenylnitril; Amide wie Dimethylformamid (DMF) oder N-methylpyrrolidon (NMP) oder Mischungen solcher Lösungsmittel, wobei Wasser, Acetonitril, Dichlormethan und Alkohole (Ethanol) besonders gut geeignet sind.

### Schritt 3.

Die im Schritt 2 gebildeten Verbindungen der Formel (V) werden zu Anthranilsäurediamid-Derivaten der Formel (I) umgesetzt.

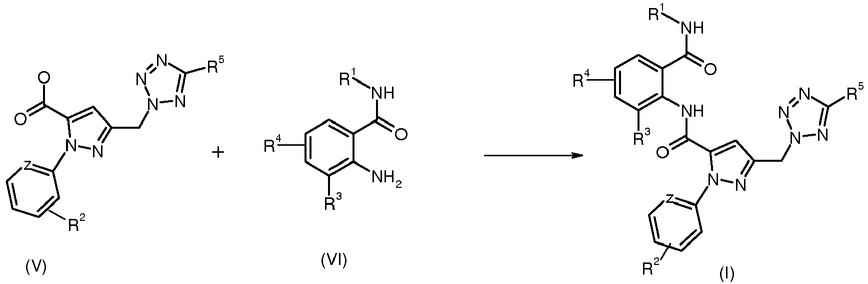

Man verwendet die Verbindungen der Formel (VI) wobei R¹ bevorzugt für (C₁-C₆) Alkyl steht.

Stufe 3 wird in Gegenwart eines Kondensationsmittels durchgeführt. Hierzu eignen sich alle für solche Kupplungsreaktionen üblichen Mittel. Beispielhaft genannt seien Säurehalogenidbildner wie Phosgen, Phosphortribromid, Phosphortrichlorid, Phosphorpentachlorid, Phosphoroxychlorid oder Thionylchlorid; Anhydridbildner wie Chlorameisensäureethylester, Chlorameisensäuremethylester, Chlorameisensäureisopropylester, Chlorameisensäureisobutylester oder Methansulfonylchlorid; Carbodiimide, wie N,N'-Dicyclohexylcarbodiimid (DCC) oder andere übliche Kondensationsmittel, wie Phosphorpentoxid, Polyphosphorsäure, 1,1'-Carbonyldiimidazol, 2-Ethoxy-N-ethoxycarbonyl-1,2-dihydrochino-lin (EEDQ), Triphenylphosphin/Tetrachlorkohlenstoff, Brom-tripyrrolidinophosphonium-hexafluorophosphat, Bis(2-oxo-3-oxazolidinyl)phosphinchlorid oder Benzotriazol-1-yloxytris(dimethylamino)-phosphonium-hexafluorphosphat. Auf Polymer gestützte Reagenzien, wie z.B. polymer-gebundenes Cyclohexylcarbodiimid, können ebenfalls verwendet werden.

Bevorzugt sind Phosgen, Mesylchlorid und Thionylchlorid.

Der Verfahrensschritt 3 kann gegebenenfalls in Gegenwart eines für solche Reaktionen üblichen inerten organischen Verdünnungsmittel durchgeführt werden. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n-oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid oder deren Gemische.

Das Verfahrensschritt 3 wird in der Regel in Gegenwart einer Base durchgeführt.

Geeignete Basen sind, Alkalimetallhydroxide wie z.B. Lithium-, Natrium- oder Kaliumhydroxid, Alkalimetallcarbonate wie beispielsweise Na₂CO₃, K₂CO₃ und -Acetate wie beispielsweise NaOAc, KOAc, LiOAc, sowie -Alkoholate, wie z.B. NaOMe, NaOEt, NaOt-Bu, KOt-Bu von organischen Basen wie Trialkylamine, Alkylpyridine, Phosphazene und 1,8-Diazabicyclo[5.4.0]undecen (DBU). Bevorzugt sind die organische Basen wie Pyridine, Alkylpyridine wie 2,6-Dimethylpyridin, 2-Methyl-5-ethylpyridin, 2,3-Dimethylpyridin.

Die Durchführung des erfindungsgemäßen Verfahrensschritts (2) erfolgt vorzugsweise innerhalb eines Temperaturbereichs von 20°C bis +100°C, besonders bevorzugt bei Temperaturen von 30°C bis +80 °C, besonders bevorzugt bei 30-60°C.

Der erfindungsgemäße Verfahrensschritt (2) wird im Allgemeinen unter Normaldruck durchgeführt. Alternativ ist es jedoch auch möglich, im Vakuum oder unter Überdruck im Autoklav zu arbeiten.

Die Reaktionszeit kann, in Abhängigkeit von der Ansatzgröße und der Temperatur, in einem Bereich zwischen 1 Stunde und mehreren Stunden gewählt werden.

Der Verfahrensschritt (3) kann gegebenenfalls in Gegenwart eines Katalysators durchgeführt werden. Beispielsweise genannt seien 4-Dimethylaminopyridin, 1-Hydroxy-benzotriazol.

Verbindungen der Formel (VI) sind bekannt oder können nach allgemeinen Synthesemethoden hergestellt werden (vgl. z. B. Baker et al. J. Org. Chem. 1952, 149-153; G. Reissenweber et al., Angew. Chem 1981, 93, 914-915, P.J. Montoya-Pelaez, J. Org. Chem. 2006, 71, 5921-5929; F. E. Sheibley, J. Org. Chem. 1938, 3, 414-423, WO 2006023783).

Verwendet man beispielsweise 2-Amino-N-tert-butyl-5-chlor-3-methylbenzamid und 1-(3-Chlorpyridin-2-yl)-3-{1-[5-(trifluormethyl)-2H-tetrazol-2-yl]ethyl}-1H-pyrazole-5-carbonsäure als Ausgangsstoffe, so kann der Verlauf des Verfahrens durch das folgende Formelschema veranschaulicht werden.

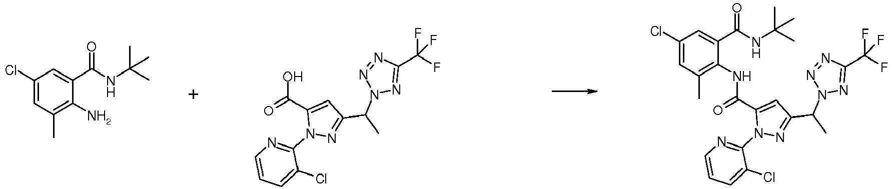

### Herstellbeispiele

Die folgenden Herstellbeispiele illustrieren die Erfindung, ohne sie zu beschränken.

### Beispiel 1

### Isomeren Gemisch von Methyl 5-(3-chloropyridin-2-yl)-3-{[5-(trifluoromethyl)-2H-tetrazol-2-yl]methyl}cyclopenta-1,3-diene-1-carboxylate (Hauptisomer) und Methyl 5-(3-chloropyridin-2-yl)-3-{[5-(trifluoromethyl)-1H-tetrazol-2-yl]methyl}cyclopenta-1,3-diene-1-carboxylate (Nebenkomponente)

2.86 g (0.01 mol) von Methyl 3-(chlormethyl)-1-(3-chloropyridin-2-yl)-1H-pyrazole-5-carboxylate und 1,6 g von Natrium 5-(trifluoromethyl)tetrazol-2-ide und 0.15 g KI in 50 ml Aceton wurden 9 Std bei 56 °C erhitzt. Die Salze wurden abfiltriert und Aceton in Vakuum entfernt. Man erhielt 4.59 g des Produktes als 9:1 Gemisch von beiden Isomeren.

### Analytische Charakterisierung

### Hauptisomer

¹H NMR (CD₃CN) δ: 8,52 (1H, d); 7.95 (1H,d), 7,45 (1H, dd); 7,10 (1H, s); 6.05 (2H,s) 3,75 (3H, s) ppm.

¹⁹F NMR -64.05 ppm.

Nebenkomponente

¹⁹F NMR -61.46 ppm.

¹H NMR (CD₃CN) δ: 8,50 (1H, d); 7.90 (1H,d), 7,45 (1H, dd); 6,95 (1H, s); 5,80 (2H,s) 3,70 (3H, s) ppm.

### Beispiel 2

### 1-(3-chloropyridin-2-yl)-3-{[5-(trifluoromethyl)-2H-tetrazol-2-yl]methyl}-1H-pyrazole-5-carboxylic acid (Hauptisomer) und 1-(3-Chloropyridin-2-yl)-3-[5-(trifluoromethyl)-1H-tetrazol-1-yl]-1H-pyrazole-5-carboxylic acid (Nebenkomponente)

4.59 g des Gemisches aus dem Beispiel 1 wurden in 40 ml Methanol gelöst und 2 g NaOH als 10 % Lösung in Wasser wurden zugegeben. Das Gemisch wurde 3 Std. bei RT gerührt.

10 % iger HCl wurde zugegeben, um den pH der Lösung auf 3 einzustellen und das Produkt wurde mit Methyltert.butylether extrahiert. Nach der Entfernung des Lösemittels wird der Rückstand (4 g) ohne Aufreinigung weiter umgesetzt.

### Analytische Charakterisierung Hauptisomer 90 %

¹H NMR (CD₃CN) δ: 13,5 (b.s) 8,52 (1H, d); 8,2 (1H,d), 7,6 (1H, dd); 7,2 (1H, s); 6.25 (2H,s) ppm.

¹⁹F NMR 64.25 - ppm.

### Beispiel 3

### Isomeren Gemisch von 1-(3-chloropyridin-2-yl)-N-[4-cyano-2-methyl-6-(methylcarbamoyl)-phenyl]-3-{[5-(trifluoromethyl)-2H-tetrazol-2-yl]methyl}-1H-pyrazole-5-carboxamide (Hauptisomer) und 1-(3-chloropyridin-2-yl)-N-[4-cyano-2-methyl-6-(methylcarbamoyl)phenyl]-3-[5-(trifluoro-methyl)-1H-tetrazol-1-yl]-1H-pyrazole-5-carboxamide (Nebenkomponente) im Verhältnis 94:6.

10 g von 1-(3-chloropyridin-2-yl)-3-{[5-(trifluoromethyl)-2H-tetrazol-2-yl]methyl}-1H-pyrazole-5-carboxylic acid und 1-(3-Chloropyridin-2-yl)-3-[5-(trifluoromethyl)-1H-tetrazol-1-yl]-1H-pyrazole-5-carboxylic acid als 90:10 Gemisch wurden in 40 ml Dimethylacetamid vorgelegt. 4.75 g von 2-Amino-5-cyano-N,3-dimethylbenzamide, 7.2 g 2,6-Dimethylpyridine und 3.9 g von Mesylchlorid wurden zugegeben und das Gemisch wurde 4 Std bei 50°C erhitzt. Das Gemisch wurde auf 20°C abgekühlt und 100 ml Wasser wurden zugegeben. Nach ca, 1 Std der Niederschlag wurde abfiltriert, mit Wasser gewaschen und gtetrocknet.. Man erhält 12.1 g des Produktes (93 % Ausbeute) mit isomeren Verhältniss 94:6. und einer Reinheit von 95-96 %.

### Analytische Charakterisierung

### Hauptisomer 94 %

| H/C | δH/ppm | Mult. | rel. No. H | δC/ppm | Mult. | rel. No. C |
|---|---|---|---|---|---|---|
| 1 | - | - | - | 118.7 | Q | 1 |
| 2 | - | - | - | 156.1 | Q | 1 |
| 3 | 6.34 | S | 2 | 51.3 | T | 1 |
| 4 | - | - | - | 145.6 | S | 1 |
| 5 | 7.40 | S | 1 | 108.5 | D | 1 |
| 6 | - | - | - | 138.8 | S | 1 |
| 7 | - | - | - | 156.3 | S | 1 |
| 8 | 10.55 | S | 1 | - | - | - |
| 9 | - | - | - | 137.6 | S | 1 |
| 10 | - | - | - | 138.7 | S | 1 |
| 11 | - | - | - | 166.2 | S | 1 |
| 12 | 8.38 | Q | 1 | - | - | - |
| 13 | 2.66 | D | 3 | 26.3 | Q | 1 |
| 14 | 7.75 | D | 1 | 129.7 | D | 1 |
| 15 | - | - | - | 109.4 | S | 1 |
| 16 | - | - | - | 118.3 | S | 1 |
| 17 | 7.87 | D | 1 | 135.2 | D | 1 |
| 18 | - | - | - | 138.0 | S | 1 |
| 19 | 2.20 | S | 3 | 18.0 | Q | 1 |
| 20 | - | - | - | 149.1 | S | 1 |
| 21 | - | - | - | 128.0 | S | 1 |
| 22 | 8.16 | DD | 1 | 139.4 | D | 1 |
| 23 | 7.60 | DD | 1 | 126.7 | D | 1 |
| 24 | 8.48 | DD | 1 | 147.3 | D | 1 |

### Nebenkomponente

| H/C | δH/ppm | Mult. | rel. No. H | δC/ppm | Mult. | rel. No. C |
|---|---|---|---|---|---|---|
| 1 | - | - | - | 118.1 | Q | 1 |
| 2 | - | - | - | 145.9 | Q | 1 |
| 3 | 6.11 | S | 2 | 47.0 | T | 1 |
| 4 | - | - | - | 145.9 | S | 1 |
| 5 | 7.35 | S | 1 | 107.7 | D | 1 |
| 6 | - | - | - | 138.8 | S | 1 |
| 7 | - | - | - | 156.2 | S | 1 |
| 8 | 10.54 | S | 1 | - | - | - |
| 9 | - | - | - | 137.6 | S | 1 |
| 10 | - | - | - | 135.2 | S | 1 |
| 11 | - | - | - | 166.2 | S | 1 |
| 12 | 8.37 | Q | 1 | - | - | - |
| 13 | 2.66 | D | 3 | 26.3 | Q | 1 |
| 14 | 7.75 | D | 1 | 129.7 | D | 1 |
| 15 | - | - | - | 109.3 | S | 1 |
| 16 | - | - | - | 118.3 | S | 1 |
| 17 | 7.87 | D | 1 | 135.4 | D | 1 |
| 18 | - | - | - | 138.0 | S | 1 |
| 19 | 2.19 | S | 3 | 17.9 | Q | 1 |
| 20 | - | - | - | 149.1 | S | 1 |
| 21 | - | - | - | 128.1 | S | 1 |
| 22 | 8.14 | DD | 1 | 139.4 | D | 1 |
| 23 | 7.58 | DD | 1 | 126.7 | D | 1 |
| 24 | 8.47 | DD | 1 | 147.2 | D | 1 |

### Beispiel 4

### 1-(3-chloropyridin-2-yl)-3-{[5-(trifluoromethyl)-2H-tetrazol-2-yl]methyl}-1H-pyrazole-5-carbonsäure (Hauptisomer) und 1-(3-Chloropyridin-2-yl)-3-[5-(trifluoromethyl)-1H-tetrazol-1-yl]-1H-pyrazole-5-carbonsäure (Nebenkomponente)

105 g (0.36 mol) von Methyl 3-(chlormethyl)-1-(3-chloropyridin-2-yl)-1H-pyrazole-5-carboxylate mit der Reinheit von 98 %, **204** g (0.384 mol) von Natrium 5-(trifluormethyl)tetrazol-2-ide (30 % Lösung in Aceton) und 24 g (0.144 mol) KI in 680 ml Aceton wurden 10 Std bei 56 °C erhitzt. Die Salze wurden abfiltriert und Aceton in Vakuum entfernt. Das Produkt (Öl) wurde in 300 ml Toluol aufgenommen und die Lösung mit 100 ml Wasser gewaschen. Die toluolische Lösung wurde dann mit 170 g 10 %iger Lösung NaOH 6 Std bei bei 40°C gerührt. Die organische Phase wurde abgetrennt und die wässrige Phase wurde mit 10 % HCl auf pH 3 langsam gestellt. Das ausgefallen Produkt wurde abfiltriert, mit Wasser gewaschen und getrocknet. Man erhielt 118 g (85 % Ausbeute) des Produktes mit einer w.w. % Reinheit von 97.3 %. Verhältnis von Regioisomeren 94:6.

Die vorliegende Erfindung betrifft weiterhin Formulierungen und daraus bereitete Anwendungsformen als Pflanzenschutzmittel und/oder Schädlingsbekämpfungsmittel wie z. B. Drench-, Drip- und Spritzbrühen. Gegebenenfalls enthalten die Anwendungsformen weitere Pflanzenschutzmittel und/oder Schädlingsbekämpfungsmittel und/oder die Wirkung verbessernde Adjuvantien wie Penetrationsförderer, z. B. vegetative Öle wie beispielsweise Rapsöl, Sonnenblumenöl, Mineralöle wie beispielsweise Paraffinöle, Alkylester vegetativer Fettsäuren wie beispielsweise Rapsöl- oder Sojaölmethylester oder Alkanol-alkoxylate und/oder Spreitmittel wie beispielsweise Alkylsiloxane und/oder Salze z.B. organische oder anorganische Ammonium- oder Phosphoniumsalze wie beispielsweise Ammoniumsulfat oder Diammonium-hydrogenphosphat und/oder die Retention fördernde Mittel wie z. B. Dioctylsulfosuccinat oder Hydroxypropyl-guar Polymere und/oder Humectants wie z.B. Glycerin und/oder Dünger wie beispielsweise Ammonium-, Kalium- oder Phosphor-enthaltende Dünger.

Übliche Formulierungen die vorliegende Erfindung betreffend sind beispielsweise Suspensionskonzentrate (SC, SE, FS, OD), in Wasser dispergierbare Granulate (WG), und Granulate (GR); diese und weitere mögliche Formuliertypen sind beispielsweise durch Crop Life International und in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576 beschrieben. Gegebenenfalls enthalten die Formulierungen neben einem oder mehreren erfindungsgemäßen Wirkstoffen weitere agrochemische Wirkstoffe.

Vorzugsweise handelt es sich um Formulierungen oder Anwendungsformen, welche Hilfsstoffe wie beispielsweise Streckmittel, Lösemittel, Spontanitätsförderer, Trägerstoffe, Emulgiermittel, Dispergiermittel, Frostschutzmittel, Biozide, Verdicker und/oder weitere Hilfsstoffe wie beispielsweise Adjuvantien enthalten. Ein Adjuvant in diesem Kontext ist eine Komponente, die die biologische Wirkung der Formulierung verbessert, ohne dass die Komponente selbst eine biologische Wirkung hat. Beispiele für Adjuvantien sind Mittel, die die Retention, das Spreitverhalten, das Anhaften an der Blattoberfläche oder die Penetration fördern.

Diese Formulierungen werden in bekannter Weise hergestellt, z.B. durch Vermischen der Wirkstoffe mit Hilfsstoffen wie beispielsweise Streckmitteln, Lösemitteln und/oder festen Trägerstoffen und/oder weiteren Hilfsstoffen wie beispielsweise oberflächenaktive Stoffe. Die Herstellung der Formulierungen erfolgt entweder in geeigneten Anlagen oder auch vor oder während der Anwendung.

Als Hilfsstoffe können solche Stoffe Verwendung finden, die geeignet sind, der Formulierung des Wirkstoffs oder den aus diesen Formulierungen bereiteten Anwendungsformen (wie z.B. gebrauchsfähigen Pflanzenschutzmitteln wie Spritzbrühen oder Saatgutbeizen) besondere Eigenschaften, wie bestimmte physikalische, technische und/oder biologische Eigenschaften zu verleihen.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (Poly-)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z.B. auch organische Lösemittel als Hilfslösemittel verwendet werden. Als flüssige Lösemittel kommen im Wesentlichen infrage: Aromaten wie Xylol, Toluol oder Alkylnaphthaline, chlorierte Aromaten oder chlorierte aliphatische Kohlenwasserstoffe wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole wie Butanol oder Glykol sowie deren Ether und Ester, Ketone wie Aceton, Methylethylketon, Methylisobutylketon oder Cyclohexanon, stark polare Lösemittel wie Dimethylformamid und Dimethylsulfoxid sowie Wasser.

Grundsätzlich können alle geeigneten Trägerstoffe eingesetzt werden. Als Trägerstoffe kommen insbesondere infrage: z.B. Ammoniumsalze und natürliche Gesteinsmehle wie Kaoline, Tonerden, Talkum, Kreide, Quarz, Attapulgit, Montmorillonit oder Diatomeenerde und synthetische Gesteinsmehl, wie hochdisperse Kieselsäure, Aluminiumoxid und natürliche oder synthetische Silikate, Harze, Wachse und /oder feste Düngemittel. Mischungen solcher Trägerstoffe können ebenfalls verwendet werden. Als Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Papier, Kokosnussschalen, Maiskolben und Tabakstängel.

Beispiele für Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, mit substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist vorteilhaft, wenn einer der Wirkstoff und/oder einer der inerten Trägerstoffe nicht in Wasser löslich ist und wenn die Anwendung in Wasser erfolgt.

Als weitere Hilfsstoffe können in den Formulierungen und den daraus abgeleiteten Anwendungsformen Farbstoffe wie anorganische Pigmente, z.B. Eisenoxid, Titanoxid, Ferrocyanblau und organische Farbstoffe wie Alizarin-, Azo- und Metallphthalocyaninfarbstoffe und Nähr- und Spurennährstoffe wie Salze von Eisen, Mangan, Bor, Kupfer, Kobalt, Molybdän und Zink vorhanden sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel. Weiterhin enthalten sein können schaumerzeugende Mittel oder Entschäumer.

Ferner können die Formulierungen und daraus abgeleiteten Anwendungsformen als zusätzliche Hilfsstoffe auch Haftmittel wie Carboxymethylcellulose, natürliche und synthetische pulverige, körnige oder latexförmige Polymere enthalten wie Gummiarabikum, Polyvinylalkohol, Polyvinylacetat sowie natürliche Phospholipide wie Kephaline und Lecithine und synthetische Phospholipide. Weitere Hilfsstoffe können mineralische und vegetabile Öle sein.

Gegebenenfalls können noch weitere Hilfsstoffe in den Formulierungen und den daraus abgeleiteten Anwendungsformen enthalten sein. Solche Zusatzstoffe sind beispielsweise Duftstoffe, schützende Kolloide, Bindemittel, Klebstoffe, Verdicker, thixotrope Stoffe, Penetrationsförderer, Retentionsförderer, Stabilisatoren, Sequestiermittel, Komplexbildner, Humectans, Spreitmittel. Im Allgemeinen können die Wirkstoffe mit jedem festen oder flüssigen Zusatzstoff, welches für Formulierungszwecke gewöhnlich verwendet wird, kombiniert werden.

Als Retentionsförderer kommen alle diejenigen Substanzen in Betracht, die die dynamische Oberflächenspannung verringern wie beispielsweise Dioctylsulfosuccinat oder die die Visko-Elastizität erhöhen wie beispielsweise Hydroxypropyl-guar Polymere.

Als Penetrationsförderer kommen im vorliegenden Zusammenhang alle diejenigen Substanzen in Betracht, die üblicherweise eingesetzt werden, um das Eindringen agrochemischer Wirkstoffen in Pflanzen zu verbessern. Penetrationsförderer werden in diesem Zusammenhang dadurch definiert, dass sie aus der (in der Regel wässerigen) Applikationsbrühe und/oder aus dem Spritzbelag in die Kutikula der Pflanze eindringen und dadurch die Stoffbeweglichkeit (Mobilität) der Wirkstoffe in der Kutikula erhöhen können. Die in der Literatur (Baur et al., 1997, Pesticide Science 51, 131-152) beschriebene Methode kann zur Bestimmung dieser Eigenschaft eingesetzt werden. Beispielhaft werden genannt Alkoholalkoxylate wie beispielsweise Kokosfettethoxylat (10) oder Isotridecylethoxylat (12), Fettsäureester wie beispielsweise Rapsöl- oder Sojaölmethylester, Fettamine Alkoxylate wie beispielsweise Tallowamine ethoxylat (15) oder Ammonium- und/oder Phosphonium-Salze wie beispielsweise Ammoniumsulfat oder Diammonium-hydrogenphosphat.

Die Formulierungen enthalten bevorzugt zwischen 0,00000001 und 98 Gew.-% Wirkstoff oder, besonders bevorzugt zwischen 0,01 und 95 Gew.-% Wirkstoff, besonders bevorzugt zwischen 0,5 und 90 Gew.-% Wirkstoff, bezogen auf das Gewicht der Formulierung.

Der Wirkstoffgehalt der aus den Formulierungen bereiteten Anwendungsformen (Pflanzenschutzmittel) kann in weiten Bereichen variieren. Die Wirkstoffkonzentration der Anwendungsformen kann üblicherweise zwischen 0,00000001 und 95 Gew.-% Wirkstoff, vorzugsweise zwischen 0,00001 und 1 Gew.-%, bezogen auf das Gewicht der Anwendungsform, liegen. Die Anwendung geschieht in einer den Anwendungsformen angepaßten üblichen Weise.

Die folgenden Beispiele illustrieren die Erfindung ohne sie einzuschränken. Die in folgenden Beispielen eingesetzten Lösungsmittelsysteme sind besonders bevorzugt. Die erfindungsgemäße Verbindung der Formel (1-1) wurde in drei verschiedenen Formulier-Rezepturen mit drei unterschiedlichen Konzentrationen zu wasserbasierten Suspensionskonzentraten verarbeitet (Formulierbeispiele 1, 2 und 3). Dabei wurde die Verbindung der Formel (I-1) als Wirkstoff sowohl in der Form A als auch in der Form B eingesetzt. Anschließend wurden die hergestellten Suspensionskonzentrate unter gleichen Bedingungen gelagert und nach Ende der Lagerung auf ihre Eigenschaften hin untersucht. Überraschenderweise wurde gefunden, dass die mit der Verbindung der Formel (1-1) als Form B hergestellten Suspensionskonzentrate sich in ihren Eigenschaften erheblich von den Suspensionskonzentraten unterschieden, die mit der Verbindung der Formel (1-1) als Form A hergestellt worden waren.

### Formulierbeispiel

### Beispiel 1

### Zur Herstellung eines Suspensionskonzentrates werden bei Raumtemperatur

| | | |
|---|---|---|
| 7,28 | Gew.-% | Verbindung (1-1) in Form A |
| 0,20 | Gew.-% | Wässrige Siliconöl-Emulsion, z.B. Silfoam SRE (Fa. Wacker) |
| 0,20 | Gew.-% | Zitronensäure |
| 0,12 | Gew.-% | Wässrige Benzoisothiazolinon-Lösung, z.B. Proxel GXL 20% |
| 0,08 | Gew.-% | Gemisch aus Bioziden vom Isothiazolon-Typ in wässriger Lösung, z.B. Preventol D7 |
| 0,50 | Gew.-% | Natrium-Alkylnaphthalinsulfonat-Formaldehydkondensat, z.B. Morwet D-425 (Fa. Akzo-Nobel) |
| 10,00 | Gew.-% | Glycerin |
| 4,00 | Gew.-% | Ethoxyliertes Polymethacrylat, z.B. Atlox 4913 (Fa. Croda) |
| 1,50 | Gew.-% | Tristyrylphenolethoxylat, z.B. Tanemul PS 54 (Fa. Tanatex) |
| 56,02 | Gew.-% | entmineralisiertes Wasser |

zusammengegeben, und nach beendeter Zugabe wird noch 10 Minuten bei Raumtemperatur nachgerührt. Die dabei entstehende homogene Suspension wird zunächst einer Grob- und dann einer Feinmahlung unterworfen, so dass eine Suspension erhalten wird, in der 90 % der Feststoffpartikel eine Teilchengröße unter 6 µm aufweisen. Danach wird mit

| | | |
|---|---|---|
| 0,10 | Gew.-% | Xanthan-Verdicker, z.B. Kelzan S (Fa. CP Kelco) |
| 20,00 | Gew.-% | entmineralisiertes Wasser |

aufgefüllt; dabei wird das Kelzan S als 2%ige Vormischung eingesetzt. Anschließend wird die Suspension solange nachgerührt bis eine homogene Suspension entstanden ist.

Analog der oben genannten Versuchsvorschrift wird ein Suspensionskonzentrat hergestellt die die Verbindung (1-1) in der Form B enthält.

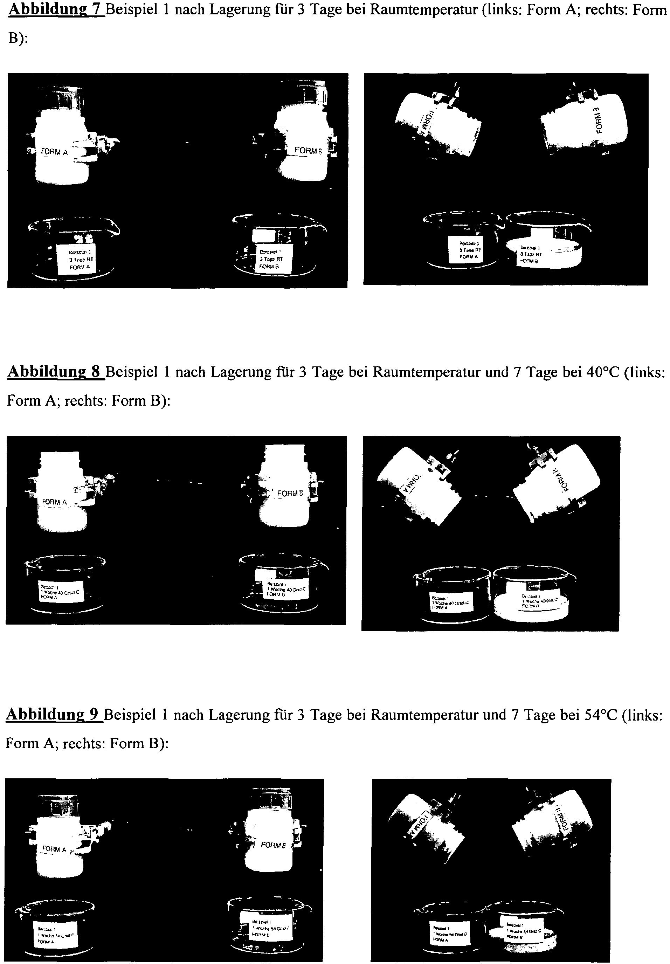

Beide Suspensionskonzentrate (Form A und Form B) sind direkt nach Herstellung homogen und dünnflüssig. Teilmengen beider Suspensionskonzentrate werden abgefüllt und zunächst 3 Tage bei Raumtemperatur stehen gelassen und danach für 7 bzw. 14 Tage bei 40°C und 54°C gelagert. Anschließend werden die Probenflaschen auf Raumtemperatur gebracht, ggf. durch leichtes Schütteln homogenisiert und verglichen (siehe Abbildung 7-10).

### Beispiel 2

### Zur Herstellung eines Suspensionskonzentrates werden bei Raumtemperatur

| | | |
|---|---|---|
| 8,47 | Gew.-% | Verbindung (1-1) in Form A |
| 0,20 | Gew.-% | Wässrige Siliconöl-Emulsion, z.B. Silfoam SRE |
| 0,20 | Gew.-% | Zitronensäure |
| 0,12 | Gew.-% | Wässrige Benzoisothiazolinon-Lösung, z.B. Proxel GXL 20% |
| 0,08 | Gew.-% | Gemisch aus Bioziden vom Isothiazolon-Typ in wässriger Lösung, z.B. Preventol D7 |
| 0,50 | Gew.-% | Natrium-Alkylnaphthalinsulfonat-Formaldehydkondensat, z.B. Morwet D-425 |
| 10,00 | Gew.-% | 1,2-Propandiol |
| 1,00 | Gew.-% | EO-PO-Block-Copolymerisat, z.B. Synperonic PE/F 127 (Fa. Croda) |
| 4,00 | Gew.-% | Tristyrylphenolethoxylat-Sulfat, z.B. Soprophor 4D384 (Fa. Rhodia) |
| 0,50 | Gew.-% | Amorphe gefällte Kieselsäure, z.B. Sipernat 22S (Fa. Evonik) |
| 54,83 | Gew.-% | entmineralisiertes Wasser |

Zusammengegeben, und nach beendeter Zugabe wird noch 10 Minuten bei Raumtemperatur nachgerührt. Die dabei entstehende homogene Suspension wird zunächst einer Grob- und dann einer Feinmahlung unterworfen, so dass eine Suspension erhalten wird, in der 90 % der Feststoffpartikel eine Teilchengröße unter 6 µm aufweisen. Danach wird mit

| | | |
|---|---|---|
| 0,10 | Gew.-% | Xanthan-Verdicker, z.B. Kelzan S |
| 20,00 | Gew.-% | entmineralisiertes Wasser |

Aufgefüllt; dabei wird das Kelzan S als 2%ige Vormischung eingesetzt. Anschließend wird die Suspension solange nachgerührt bis eine homogene Suspension entstanden ist.

Analog der oben genannten Versuchsvorschrift wird ein Suspensionskonzentrat hergestellt die die Verbindung (1) in der Form B enthält.

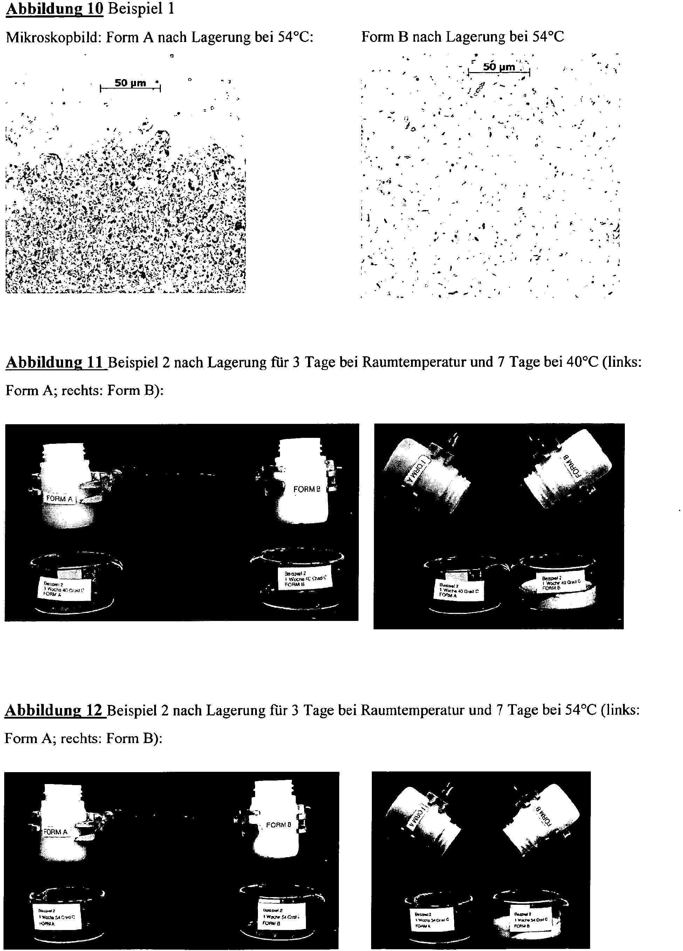

Beide Suspensionskonzentrate (Form A und Form B) sind direkt nach Herstellung homogen und dünnflüssig. Teilmengen beider Suspensionskonzentrate werden abgefüllt und zunächst 3 Tage bei Raumtemperatur stehen gelassen und danach für 7 bzw. 14 Tage bei 40°C und 54°C gelagert. Anschließend werden die Probenflaschen auf Raumtemperatur gebracht, ggf. durch leichtes Schütteln homogenisiert und verglichen (siehe Abbildung 11-13).

### Beispiel 2 nach Lagerung für 3 Tage bei Raumtemperatur:

Nach 3 Tagen Lagerung bei Raumtemperatur sind beide Proben homogen und dünnflüssig.

### Beispiel 3

### Zur Herstellung eines erfindungsgemäßen Suspensionskonzentrates werden bei Raumtemperatur

| | | |
|---|---|---|
| 4,85 | Gew.-% | Verbindung (1-1) in Form A |
| 0,20 | Gew.-% | Wässrige Siliconöl-Emulsion, z.B. Silfoam SRE |
| 0,20 | Gew.-% | Zitronensäure |
| 0,12 | Gew.-% | Wässrige Benzoisothiazolinon-Lösung, z.B. Proxel GXL 20% |
| 0,08 | Gew.-% | Gemisch aus Bioziden vom Isothiazolon-Typ in wässriger Lösung, z.B. Preventol D7 |
| 1,50 | Gew.-% | Natrium-Alkylnaphthalinsulfonat-Formaldehydkondensat, z.B. Morwet D-425 |
| 10,00 | Gew.-% | 1,2-Propandiol |
| 6,25 | Gew.-% | Ethoxyliertes Polymethacrylat, z.B. Atlox 4913 |
| 2,00 | Gew.-% | Butyl-EO-PO-Block-Copolymer, z.B. Atlas G-5000 (Fa. Croda) |
| 54,60 | Gew.-% | entmineralisiertes Wasser |

Zusammengegeben, und nach beendeter Zugabe wird noch 10 Minuten bei Raumtemperatur nachgerührt. Die dabei entstehende homogene Suspension wird zunächst einer Grob- und dann einer Feinmahlung unterworfen, so dass eine Suspension erhalten wird, in der 90 % der Feststoffpartikel eine Teilchengröße unter 6 µm aufweisen. Danach wird mit

| | | |
|---|---|---|
| 0,20 | Gew.-% | Xanthan-Verdicker, z.B. Kelzan S |
| 20,00 | Gew.-% | entmineralisiertes Wasser |

Aufgefüllt; dabei wird das Kelzan S als 2%ige Vormischung eingesetzt. Anschließend wird die Suspension solange nachgerührt bis eine homogene Suspension entstanden ist.

Analog der oben genannten Versuchsvorschrift wird ein Suspensionskonzentrat hergestellt die die Verbindung (1-1) in der Form B enthält.

Beide Suspensionskonzentrate (Form A und Form B) sind direkt nach Herstellung homogen und flüssig. Teilmengen beider Suspensionskonzentrate werden abgefüllt und zunächst 3 Tage bei Raumtemperatur stehen gelassen und danach für 7 bzw. 14 Tage bei 40°C und 54°C gelagert. Anschließend werden die Probenflaschen auf Raumtemperatur gebracht und verglichen.

### Beispiel 3 nach Lagerung für 3 Tage bei Raumtemperatur:

Nach 3 Tagen Lagerung bei Raumtemperatur sind beide Proben homogen und dünnflüssig

### Beispiel 3 nach Lagerung für 3 Tage bei Raumtemperatur und 7 Tage bei 40°C bzw. 54°C:

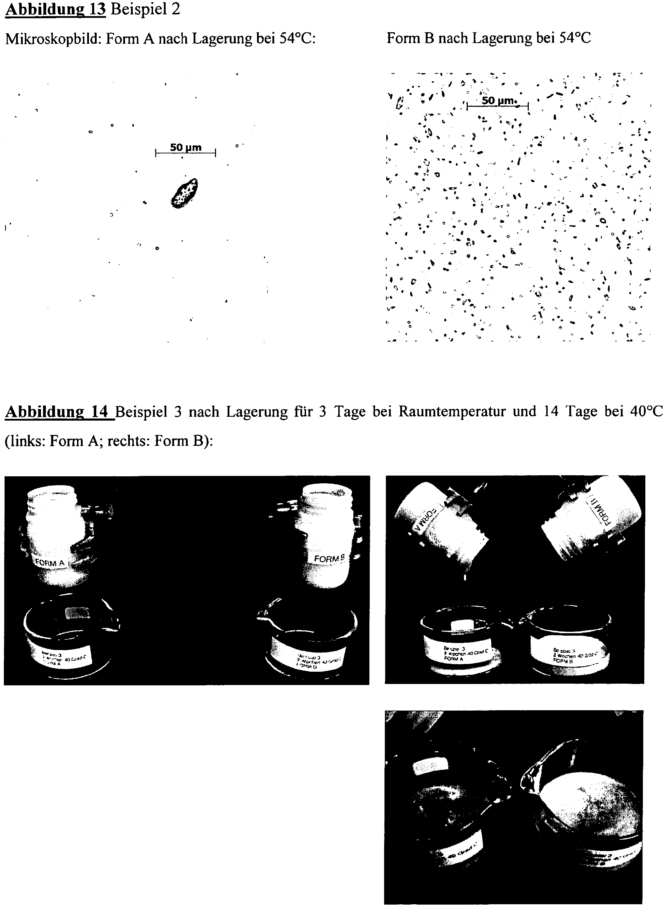

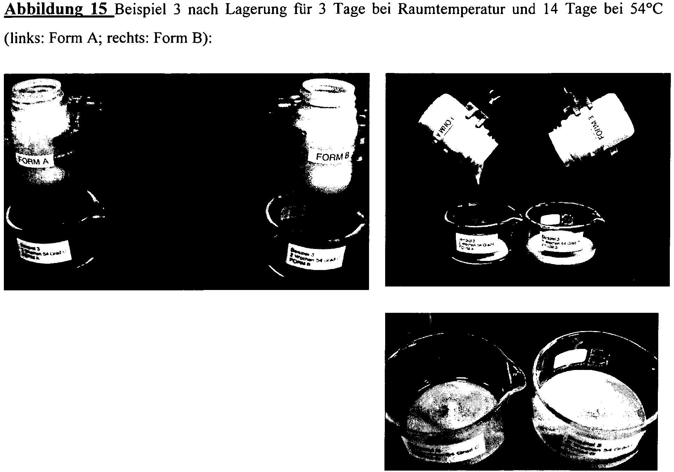

Nach 3 Tagen Lagerung bei Raumtemperatur und 7 Tagen bei 40°C bzw. 54°C sind beide Proben dünnflüssig, zeigen aber eine leichte Phasentrennung (siehe Abbildung 14-15).

**Tabelle 1: Röntgenpulverdiffraktometrie**

| **Reflexe [2 Theta]** | |
|---|---|
| **Modifikation A** | **Modifikation B** |
| 2.9 | 5.7 |
| 5.2 | 6.4 |
| 5.9 | 7.3 |
| 6.5 | 8.3 |
| 7.0 | 9.1 |
| 7.3 | 9.6 |
| 7.4 | 10.2 |
| 8.1 | 10.6 |
| 9.6 | 11.4 |
| 10.6 | 11.7 |
| 11.6 | 12.8 |
| 12.3 | 14.9 |
| 13.1 | 16.2 |
| 14.2 | 16.8 |
| 15.9 | 17.6 |
| 18.8 | 18.2 |
| 19.6 | 18.9 |
| 20.3 | 19.3 |
| 21.0 | 19.7 |
| 22.1 | 20.0 |
| 22.7 | 20.5 |
| 23.1 | 21.1 |
| 23.8 | 21.5 |
| 24.3 | 21.8 |
| 25.4 | 22.2 |
| 25.8 | 22.9 |
| 26.9 | 23.2 |
| 27.5 | 23.4 |
| 31.3 | 23.5 |
| 32.4 | 24.0 |
| 37.2 | 24.3 |
| | 24.6 |
| | 25.2 |
| | 25.8 |
| | 25.8 |
| | 27.3 |
| | 27.8 |
| | 28.0 |
| | 28.3 |
| | 29.4 |
| | 29.7 |
| | 30.4 |
| | 31.0 |
| | 31.7 |
| | 32.2 |
| | 32.9 |
| | 34.3 |
| | 35.0 |
| | 37.1 |

**Tabelle 2: IR- und Raman-Banden**

| ***Modifikation A*** | ***Modifikation B*** | ***Modifikation A*** | ***Modifikation B*** |
|---|---|---|---|
| **IR-Banden [cm⁻¹]** | **IR-Banden [cm⁻¹]** | **Raman-Banden [cm⁻¹]** | **Raman-Banden [cm⁻¹]** |
| 3238 | 3280 | 3117 | 3073 |
| 3087 | 3002 | 3070 | 2997 |
| 2232 | 2977 | 2962 | 2929 |
| 1662 | 2233 | 2927 | 2881 |
| 1636 | 1661 | 2235 | 2231 |
| 1579 | 1637 | 2231 | 2236 |
| 1541 | 1602 | 1664 | 1664 |
| 1510 | 1574 | 1601 | 1602 |
| 1468 | 1543 | 1578 | 1574 |
| 1410 | 1510 | 1567 | 1543 |
| 1379 | 1466 | 1543 | 1512 |
| 1332 | 1446 | 1516 | 1464 |
| 1304 | 1421 | 1468 | 1424 |
| 1275 | 1410 | 1419 | 1386 |
| 1249 | 1385 | 1378 | 1335 |
| 1220 | 1334 | 1332 | 1275 |
| 1155 | 1298 | 1306 | 1244 |
| 1080 | 1275 | 1277 | 1229 |
| 1054 | 1244 | 1252 | 1219 |
| 1022 | 1219 | 1218 | 1141 |
| 930 | 1178 | 1147 | 1081 |
| 887 | 1155 | 1081 | 1044 |
| 797 | 1080 | 1048 | 1022 |
| 773 | 1055 | 1021 | 1008 |
| 751 | 1046 | 966 | 897 |
| 736 | 1029 | 929 | 882 |
| 690 | 1023 | 889 | 772 |
| | 1009 | 813 | 760 |
| | 954 | 775 | 745 |
| | 902 | 744 | 636 |
| | 882 | 645 | 539 |
| | 829 | 594 | 489 |
| | 803 | 537 | 475 |
| | 795 | 489 | 457 |
| | 774 | 474 | 350 |
| | 758 | 449 | 337 |
| | 743 | 434 | 327 |
| | 729 | 400 | 296 |
| | 669 | 331 | 271 |
| | | 274 | 218 |
| | | 245 | 115 |
| | | 129 | |
| | | 100 | |
| | | 84 | |

## Patentansprüche

1. Verfahren zur Herstellung der kristallinen Modifikation B der Verbindung der Formel (1-1) 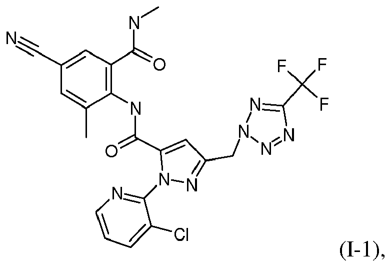 **dadurch gekennzeichnet, dass** man N-Aryl und N-hetarylsubstituierte Pyrazole der Formel (II) 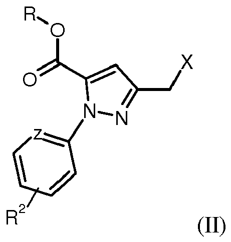 in welcher
R für (C₁-C₆) Alkyl, Aryl(C₁-C₆)-Alkyl oder Aryl steht,
R² für Chlor steht,
Z für N steht,
und X für Fluor, Chlor, Brom, Jod, CH₃SO₂O, CF₃SO₃ oder p-CH₃-C₆H₄SO₃ steht, mit Tetrazolen der Formel (III) 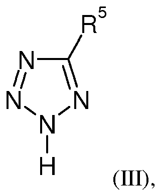 zu Pyrazolcarbonsäureestern der Formel (IV) umsetzt 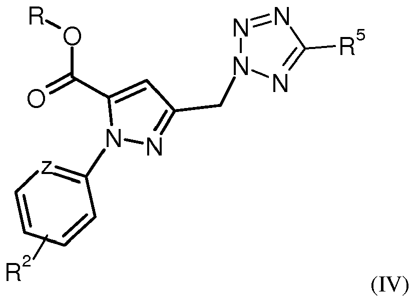 in welcher
R, R², Z, die oben angegebenen Bedeutungen haben und R⁵ für CF₃ steht,
und diese gegebenenfalls ohne vorherige Isolierung zu Pyrazolcarbonsäuren der Formel (V) umsetzt 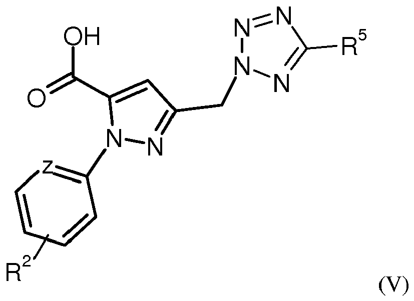 in welcher R², R⁵ und Z die oben angegebenen Bedeutungen haben
und diese mit Verbindungen der allgemeinen Formel (VI) 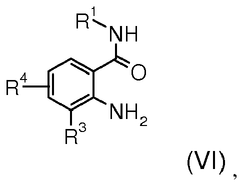 in welcher
R¹, R³ für Methyl steht und R⁴ für Cyano steht,
umsetzt.

2. Kristalline Modifikation B der Verbindung der Formel (1-1) 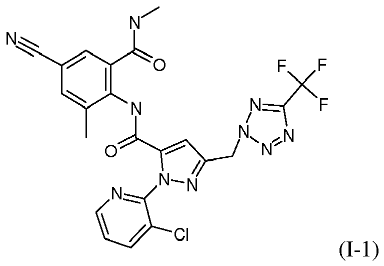 **dadurch gekennzeichnet, dass** ihr Röntgen-Pulverdiffraktogramm bei Verwendung von Cu Kα Strahlung mindestens folgende Reflexlagen aufweist:
| **2Theta / °** |
|---|
| 5.7 |
| 6.4 |
| 11.4 |
| 17.6 |
| 18.9 |
| 21.1 |
| 23.2 |
| 23.4 |
| 23.5 |
| 25.2 |

3. Kristalline Modifikation B gemäß Anspruch 2, **dadurch gekennzeichnet, dass** ihr Röntgen-Pulverdiffraktogramm bei Verwendung von Cu Kα-Strahlung mindestens folgende weitere Reflexlagen aufweist:
| **2Theta / °** |
|---|
| 11.7 |
| 16.8 |
| 19.7 |
| 20.5 |
| 24.3 |
| 24.6 |
| 28.3 |

4. Kristalline Modifikation B der Verbindung der Formel (1-1) gemäß einem oder mehreren der Ansprüche 2 bis 3, **dadurch gekennzeichnet, dass** ihr Raman-Spektrum mindestens folgende Banden aufweist:
| **Bande [cm⁻¹]** |
|---|
| 2929 |
| 1386 |
| 882 |
| 636 |

5. Agrochemische Zusammensetzung umfassend die Verbindung der Formel (1-1) der kristallinen Modifikation B nach einem oder mehreren der Ansprüche 2 bis 4.

6. Verwendung der Verbindung der Formel (1-1) der kristallinen Modifikation B nach einem oder mehreren der Ansprüche 2 bis 4 zur Herstellung von insektizid wirksamen Zusammensetzungen.

7. Verfahren zur Bekämpfung von unerwünschten Insekten-ausgenommen zur therapeutischen Behandlung des tierischen oder menschlichen Körpers-, **dadurch gekennzeichnet, dass** eine agrochemische Zusammensetzung umfassend die Verbindung der Formel (1-1) der kristallinen Modifikation B gemäß einem oder mehreren der Ansprüche 2 bis 4 die Insekten und/oder ihren Lebensraum und/oder Saatgut ausgebracht wird.

## Claims

1. Process for preparing the crystal polymorph B of the compound of the formula (I-1) 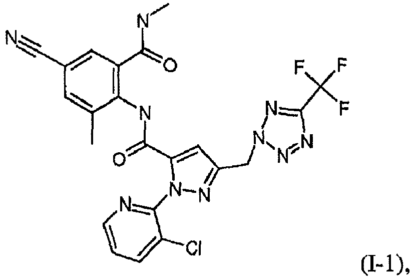 **characterized in that** N-aryl- and N-hetaryl-substituted pyrazoles of the formula (II) 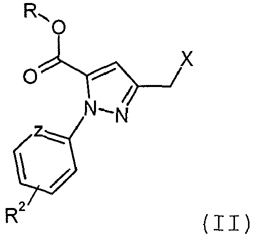 in which
R is C₁-C₆-alkyl, aryl(C₁-C₆)alkyl or aryl
R² is chlorine
Z is N
and X is fluorine, chlorine, bromine, iodine, CH₃SO₂O, CF₃SO₃, or p-CH₃-C₆H₄SO₃,
are reacted with tetrazoles of the formula (III) 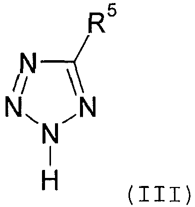 to give pyrazolecarboxylic esters of the formula (IV) 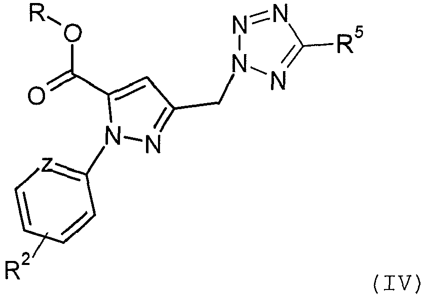 in which
R, R² and Z are each as defined above and R⁵ is CF₃,
and the latter are optionally converted without preceding isolation to pyrazolecarboxylic acids of the formula (V) 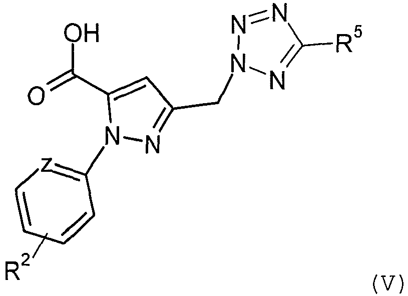 in which R², R⁵ and Z are each as defined above
and the latter are reacted with compounds of the general formula (VI) 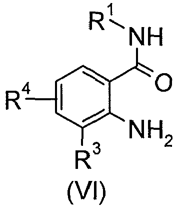 in which R¹, R³ are each methyl and R⁴ is cyano.

2. Crystal polymorph B of the compound of the formula (I-1) 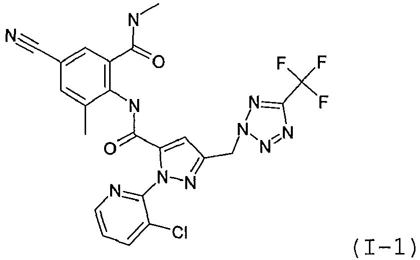 **characterized in that** the x-ray powder diffractogram thereof using Cu Kα radiation has at least the following reflections:
| **2theta / °** |
|---|
| 5.7 |
| 6.4 |
| 11.4 |
| 17.6 |
| 18.9 |
| 21.1 |
| 23.2 |
| 23.4 |
| 23.5 |
| 25.2 |

3. Crystal polymorph B according to Claim 2, **characterized in that** the x-ray powder diffractogram thereof using Cu Kα radiation has at least the following further reflections:
| **2theta /** ° |
|---|
| 11.7 |
| 16.8 |
| 19.7 |
| 20.5 |
| 24.3 |
| 24.6 |
| 28.3 |

4. Crystal polymorph B of the compound of the formula (I-1) according to one or more of Claims 2 to 3, **characterized in that** the Raman spectrum thereof has at least the following bands:
| **Band [cm⁻¹]** |
|---|
| 2929 |
| 1386 |
| 882 |
| 636 |

5. Agrochemical composition comprising the compound of the formula (I-1) of crystal polymorph B according to one or more of Claims 2 to 4.

6. Use of the compound of the formula (I-1) of crystal polymorph B according to one or more of Claims 2 to 4 for production of insecticidally active compositions.

7. Method for controlling unwanted insects, excluding therapeutic treatment of the animal or human body, **characterized in that** an agrochemical composition comprising the compound of the formula (I-1) of crystal polymorph B according to one or more of Claims 2 to 4 is applied to the insects and/or their habitat and/or seed.

## Revendications

1. Procédé pour la préparation de la forme cristalline B du composé de formule (I-1) 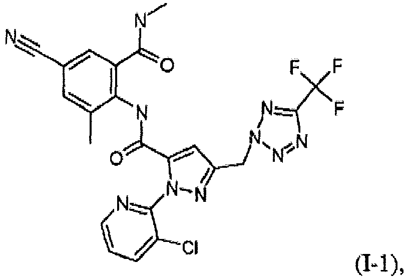 **caractérisé en ce qu'**on fait réagir des pyrazoles N-aryl- et N-hétéroaryl-substitués de formule (II) 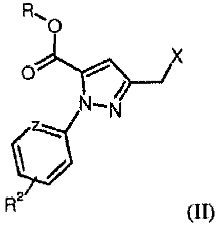 dans laquelle
R représente un groupe alkyle en C₁-C₆, aryl-alkyle(C₁-C₆) ou aryle,
R² représente un atome de chlore,
Z représente N,
et X représente un atome de fluor, de chlore, de brome, d'iode, CH₃SO₂O, CF₃SO₃ ou p-CH₃-C₆H₄SO₃,
avec des tétrazoles de formule (III) 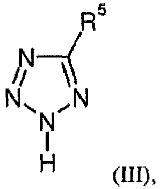 pour aboutir à des esters d'acides pyrazole-carboxyliques de formule (IV) 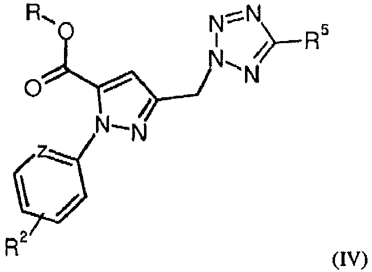 dans laquelle
R, R², Z ont les significations indiquées plus haut et R⁵ représente CF₃,
et on convertit éventuellement ces derniers, sans isolement préalable, en acides pyrazolecarboxyliques de formule (V) 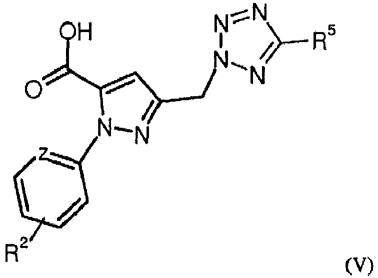 dans laquelle R², R⁵ et Z ont les significations indiquées plus haut
et on fait réagir ces derniers avec des composés de formule générale (VI) 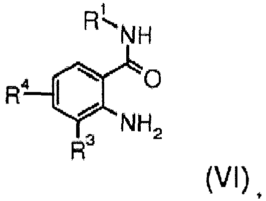 dans laquelle
R¹, R³ représentent le groupe méthyle et R⁴ représente le groupe cyano.

2. Forme cristalline B du composé de formule (I-1) 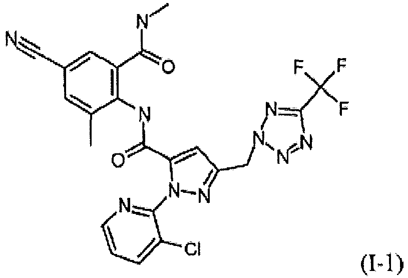 **caractérisée en ce que** son diffractogramme aux rayons X sur poudre avec utilisation de la raie Kα du cuivre comporte au moins les positions de réflexions suivantes :
| **2 thêta/°** |
|---|
| 5,7 |
| 6,4 |
| 11,4 |
| 17,6 |
| 18,9 |
| 21,1 |
| 23,2 |
| 23,4 |
| 23,5 |
| 25,2 |

3. Forme cristalline B selon la revendication 2, **caractérisée en ce que** son diffractogramme aux rayons X sur poudre avec utilisation de la raie Kα du cuivre comporte au moins les autres positions de réflexions suivantes :
| **2 thêta/°** |
|---|
| 11,7 |
| 16,8 |
| 19,7 |
| 20,5 |
| 24,3 |
| 24,6 |
| 28,3 |

4. Forme cristalline B du composé de formule (I-1) selon une ou plusieurs des revendications 2 et 3, **caractérisée en ce que** son spectre Raman comporte au moins les bandes suivantes :
| **Bande [cm⁻¹]** |
|---|
| 2929 |
| 1386 |
| 882 |
| 636 |

5. Composition agrochimique comprenant le composé de formule (I-1) de forme cristalline B selon une ou plusieurs des revendications 2 à 4.

6. Utilisation du composé de formule (I-1) de forme cristalline B selon une ou plusieurs des revendications 2 à 4, pour la préparation de compositions à activité insecticide.

7. Procédé pour la lutte contre des insectes indésirables - à l'exclusion du traitement thérapeutique du corps humain ou animal -, **caractérisé en ce qu'**on épand su les insectes et/ou leur habitat et/ou des semences une composition agrochimique comprenant le composé de formule (I-1) de forme cristalline B selon une ou plusieurs des revendications 2 à 4.
